(19) Europäisches Patentamt
European Patent Office
Office européen des brevets

(11) Publication number : **0 507 488 A1**

(12) # EUROPEAN PATENT APPLICATION

(21) Application number : **92302531.6**

(22) Date of filing : **24.03.92**

(51) Int. Cl.$^5$ : **C07D 213/64,** C07D 307/81, C07C 211/48, A61K 31/44, A61K 31/34

(30) Priority : **27.03.91 US 676031**

(43) Date of publication of application : **07.10.92 Bulletin 92/41**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Wai, John Sui Man**
**208 Tanglewood Way**
**Harleysville, Pennsylvania 19438 (US)**
Inventor : **Saari, Walfred S.**
**1740 Wagon Wheel Lane**
**Lansdale, Pennsylvania 19446 (US)**
Inventor : **Holloway, Mary Katharine**
**171 Forest Trail Drive**
**Lansdale, Pennsylvania 19446 (US)**

(74) Representative : **Thompson, John Dr. et al**
**Merck & Co., Inc. European Patent Department Terlings Park Eastwick Road**
**Harlow, Essex CM20 2QR (GB)**

(54) **Inhibitors of HIV-1 reverse transcriptase.**

(57) Novel aniline derivatives containing hydroxy and hydroxymethyl groups inhibit HIV-1 reverse transcriptase, and are useful in the prevention or treatment of infection by HIV-1 and the treatment of AIDS, either as compounds, pharmaceutically acceptable salts, pharmaceutical composition ingredients, whether or not in combination with other antivirals, anti-infectives, immunomodulators, antibiotics or vaccines. Methods of treating AIDS and methods of preventing or treating infection by HIV-1 are also described.

EP 0 507 488 A1

Jouve, 18, rue Saint-Denis, 75001 PARIS

The present invention is concerned with compounds which inhibit the reverse transcriptase encoded by human immunodeficiency virus (HIV) or pharmaceutically acceptable salts thereof and are of value in the prevention of infection by HIV, the treatment of infection by HIV-1 and the treatment of the resulting acquired immune deficiency syndrome (AIDS). It also relates to pharmaceutical compositions containing the compounds and to a method of use of the present compounds and other agents for the treatment of AIDS & viral infection by HIV.

## BACKGROUND OF THE INVENTION

A retrovirus, designated human immunodeficiency virus (HIV), is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is reverse transcription of the RNA genome by a virally encoded reverse transcriptase to generate DNA copies of HIV-1 sequences, a required step in viral replication. It is known that some compounds are reverse transcriptase inhibitors and are effective agents in the treatment of AIDS and similar diseases, e.g., azidothymidine or AZT.

Nucleotide sequencing of HIV-1 shows the presence of a pol gene in one open reading frame [Ratner, L. et al., Nature, 313, 277(1985)]. Amino acid sequence homology provides evidence that the pol sequence encodes reverse transcriptase, an endonuclease and a HIV-1 protease [Toh, H. et al., EMBO J. 4, 1267 (1985); Power, M.D. et al., Science, 231, 1567 (1986); Pearl, L.H. et al., Nature 329, 351 (1987)].

The compounds of this invention are inhibitors of HIV-1 reverse transcriptase. The inhibition is very specific, since there is little or no inhibition of the reverse transcriptases of AMV, MMLV or SIV.

## BRIEF DESCRIPTION OF THE INVENTION

Compounds of formula I, as herein defined, are disclosed. These compounds are useful in the inhibition of HIV-1 reverse transcriptase, the prevention of infection by HIV, the treatment of infection by HIV-1 and the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV-1 are also disclosed.

## DETAILED DESCRIPTION OF THE INVENTION AND PREFERRED EMBODIMENTS

This invention is concerned with the use of compounds of formula I, combinations thereof, or pharmaceutically acceptable salts thereof, in the inhibition of HIV-1 reverse transcriptase, the prevention or treatment of infection by HIV-1 and in the treatment of the resulting acquired immune deficiency syndrome (AIDS). Compounds of formula I are defined as follows:

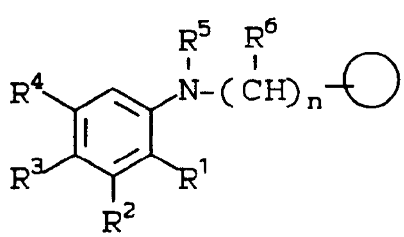

I

wherein:

n is 1-4;

$R^1$ is H, OH, or $CH_2OH$;

$R^2$ is H, or $CH_2OH$;

$R^3$ is H, $C_{1-8}$ alkyl unsubstituted or substituted with one or two of methoxy, or methylthio;

$R^4$ is

(i) $C_{1-8}$ alkyl, unsubstituted or substituted with one or two of $C_{1-3}$alkoxy, halo, or $C_{1-3}$ alkylthio;

(ii) $C_{1-3}$ alkylthio;
(iii) $C_{1-3}$ alkoxy;
(iv) halo; or
(v) hydroxy alkyl ($C_1$-$C_4$);
$R^5$ is H or $C_{1-2}$ alkyl;
$R^6$ is H or $C_{1-2}$ alkyl; and

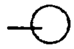 is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkylamino,
(e) di($C_{1-6}$ alkyl)amino,
(f) amino- $C_{1-3}$ alkyl,
(g) $C_{1-3}$ alkyl-amino-$C_{1-8}$ alkyl,
(h) di-($C_{1-6}$ alkyl)amino $C_{1-3}$ alkyl,
(i) hydroxyl, or
(j) halogen.

One embodiment of the present invention is Formula I compounds, wherein
$R^1$ is H or OH;
$R^2$ is H or $CH_2OH$;
$R^3$ is H or $CH_3$;
$R^4$ is $C_{1-4}$ alkyl or hydroxy alkyl (C1-C4);
$R^5$ and $R^6$ are H;

aryl is
(a) naphthyl, unsubstituted or substituted with one or more of $C_{1-6}$ alkyl, halogen, hydroxy or alkoxyl;
(b) phenyl, unsubstituted or substituted with one or more of $C_{1-6}$ alkyl, halogen, hydroxyl, or alkoxyl; or
(c) biphenyl.

A second embodiment comprises compounds wherein aryl is 2-naphthyl, 2-naphthyl substituted with methyl, 2-naphthyl substituted with chloro; 2-napthyl substituted with alkoxy; phenyl; phenyl substituted with alkoxy and between one and three alkyl groups of $C_1$-$C_6$.

A third embodiment is compounds further limited, wherein aryl is 2-naphthyl, 2-methoxy-4, 5-dimethyl phenyl, or 2-methoxy-5-ethyl phenyl.

A fourth embodiment comprises compounds of Formula 1, wherein heterocycle is thienyl, oxazolyl, thiazolyl, triazolyl, pyridyl, pyrazinyl, benzothienyl, quinolinyl, isoquinolinyl, benzimidazolyl, benzofuranyl, benzothiazolyl, benzoxazolyl unsubstituted or substituted with one or more of $C_{1-6}$ alkyl, halogen, hydroxyl, or alkoxyl naphth [3,2-d] oxazolyl, oxazolo [4,5-b] pyridyl, chromone, or benzopyrimidinone.

A fifth embodiment covers compounds having heterocycle further limited to 2-oxazolyl, 2-pyridyl, 2-benzothienyl, 2-quinolinyl, 2-benzimidazolyl, 2-benzofuranyl, 2-benzothiazolyl, 2-oxazolo [4,5-b] pyridyl, or 2-benzoxazolyl unsubstituted or substituted with one or more of methyl, halogen or alkoxyl.

A sixth embodiment covers compounds having heterocycle further limited to 2-benzoxazolyl, 7-methyl-2-benzoxazolyl, 4,7-dimethyl-2-benzoxazolyl, 7-chloro-2-benzoxazolyl, 4,7-dichloro-2-benzoxazolyl, 2-methoxy-5-ethyl-6-methyl-3-pyridyl, or 2-methoxy-5, 6-dimethyl-3-pyridyl.

Preferred compounds of this invention include, but are not limited to the following list:
4-ethyl-2-[(2-naphthylmethyl)amino]phenol;
4-ethyl-5-methyl-2-{[4,7-dichlorobenzo(b)furan-2-ylmethyl]amino}phenol;
4-ethyl-5-methyl-2-{[4,7-dimethylbenzo(b)furan-2-ylmethyl]amino}phenol;
4-ethyl-2-{[3-(5-ethyl-2-methoxy-6-methylpyridyl)methyl]amino}phenol;
5-ethyl-3-{[3-(5-ethyl-2-methoxy-6-methylpyridyl)methyl]amino}benzyl alcohol;
5-ethyl-3{[3-(hydroxymethyl)anilino]methyl}-2-methoxy-6-methylpyridine;
2-(4,5-dimethyl-2-methoxybenzylamino)-4-ethyl-5--methylphenol;
5-ethyl-2-methoxy-3-[(3-methoxyanilino)methyl]-6-methylpyridine;
5-ethyl-3-[(2-hydroxyanilino)methyl]-2-methoxy-6-methylpyridine;
5-ethyl-3-[(2-hydroxy-3-hydroxymethyl-5-methylanilino)methyl]-2-methoxy-6-methylpyridine.

The compounds of the present invention, may have asymmetric centers and occur as diastereomers, racemates, racemic mixtures, individual enantiomers, and mixtures thereof with all isomeric forms being included

in the present invention.

When any variable (e.g., aryl, heterocycle, $R^1$, $R^2$, $R^3$, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms; "alkoxy" represents an alkyl group of indicated number of carbon atoms attached through an oxygen bridge. "Halogen" or "halo" as used herein, means fluoro, chloro, bromo or iodo.

As used herein, with exceptions as noted, "aryl" is intended to mean any stable monocyclic, bicyclic or tricyclic carbon ring of up to 7 members in each ring, wherein at least one ring is aromatic. Examples of such aryl elements include phenyl, naphthyl, tetrahydronaphthyl, biphenyl, phenanthryl, anthryl or acenaphthyl.

The term heterocycle or heterocyclic, as used herein except where noted, represents a stable 5- to 7-membered monocyclic or stable 8- to 11-membered bicyclic or stable 11-15-membered tricyclic heterocyclic ring which is either saturated or unsaturated, and which consists of carbon atoms and from one to four heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, benzofuranyl, tetrahydropyranyl, thienyl, benzothienyl, thiamorpholinyl, thiamorpholinyl sulfoxide, thiamorpholinyl sulfone, and oxadiazolyl.

In the compounds of formula I, $R^1$, $R^2$, $R^3$, $R^4$, $R^5$ and $R^6$ include but are not limited to the following substituents, listed in tabular form. Examples shown have n=1.

# TABLE I

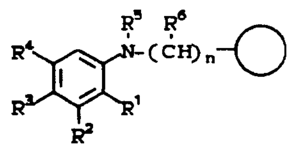

| | $-R^1$ | $-R^2$ | $-R^3$ | $-R^4$ | $-R^5$ | $-R^6$ | 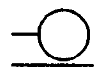 |
|---|---|---|---|---|---|---|---|
| 1) | OH | H | H | $CH_2CH_3$ | H | H | 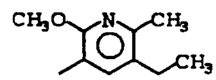 |
| 2) | OH | H | $CH_3$ | $CH_2CH_3$ | H | H | 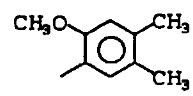 |
| 3) | OH | H | H | $CH_2CH_3$ | H | H | 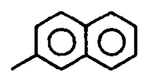 |
| 4) | OH | H | $CH_3$ | $CH_2CH_3$ | H | H | 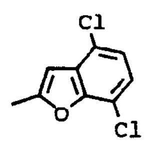 |
| 5) | H | H | H | $OCH_3$ | H | H | 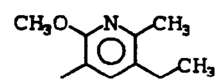 |
| 6) | OH | H | H | H | H | H | 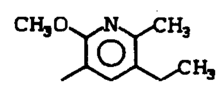 |
| 7) | H | $CH_2OH$ | H | H | H | H | 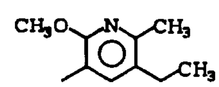 |
| 8) | OH | $CH_2OH$ | H | $CH_3$ | H | H | 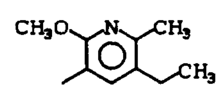 |

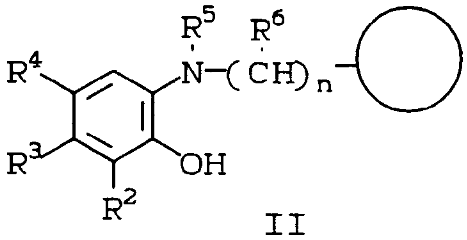

II

The synthesis of compounds of formula I is specifically shown in the examples. As species of formula I, compounds of formula II or V are further described hereinbelow.

In the synthesis of the aminophenols II of this invention, applicants here provide preferred methods outlined below.

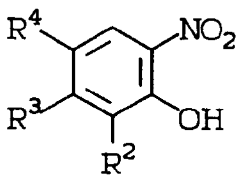

III

The 2-nitrophenols, III, of the first step in the synthesis of II may be formed by nitration of 2-unsubstituted phenols. The 2-nitrophenol products of the first step are reduced to IV

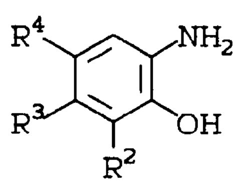

IV

in the second step, preferably by catalytic reduction (when sulfur atoms are not present) in the presence of e.g. $H_2$ gas and Pd or Pt on carbon catalyst in a solvent such as ethanol. Alternatively, the reduction of the second step (including when sulfur atoms are present) can be performed by chemical means, e.g. with NaSH, $NaS_2O_4$, Fe + HOAc, $H_2S$, or Sn + HCl. A third step, giving rise to the compounds II of this invention, involves a coupling reaction, either by alkylation with an appropriate alkylhalide or an equivalent electrophile, or by reductive alkylation with an appropriate aldehyde.

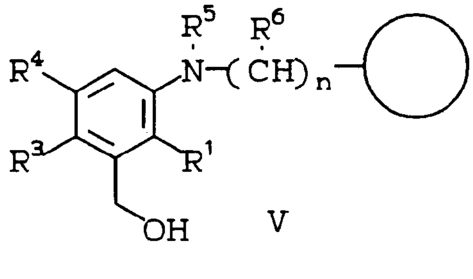

V

In the synthesis of the 3-aminobenzyl alcohols V of this invention, applicants here provide preferred methods outlined below.

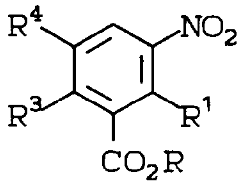

VI

The 3-nitrobenzoates, VI, of the first step in the synthesis of V may be formed by nitration of 3-unsubstituted benzoates. The 3-nitrobenzoate products of the first step are reduced to 3-aminobenzoates VII in the second step, preferably by catalytic reduction (when sulfur atoms are not present) in the presence of e.g. $H_2$ gas and Pd or Pt on carbon catalyst in a solvent such as ethanol. Alternatively, the reduction of the second step (including when sulfur atoms are present) can be performed by chemical means, e.g. with NaSH, $NaS_2O_4$, Fe + HOAc, $H_2S$, or Sn + HCl.

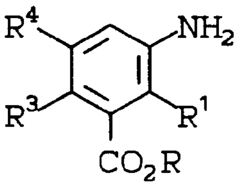

VII

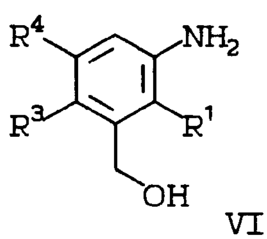

VIII

In the third step, the 3-aminobenzoates, (VII) are further reduced to 3-aminobenzyl alcohols VIII with reductants such as lithium aluminum hydride, diisopropylaluminum hydride, etc. For the final step, reductive alkylation with appropriate aldehydes or alkylation with appropriate halides, gives rise to the compounds (V) of this invention.

Appropriate alkylhalides and aldehydes are commercially available, the syntheses are known in the art, or are described in the examples provided herein. For example, the synthesis of the alkylhalide and aldehyde intermediates of this invention may follow the procedures and protocols disclosed in application USSN 539,643 or 646,131, corresponding to EP-A-0462800.

The compounds of the present inventions are useful in the inhibition of HIV-1 reverse transcriptase, the prevention or treatment of infection by the human immunodeficiency virus (HIV-1) and the treatment of consequent pathological conditions such as AIDS. Treating AIDS or preventing or treating infection by HIV-1 is defined as including, but not limited to, treating a wide range of states of HIV-1 infection: AIDS, ARC (AIDS related complex), both symptomatic and asymptomatic, and actual or potential exposure to HIV-1. For example, the compounds of this invention are useful in treating infection by HIV-1 after suspected past exposure to HIV-1 by e.g., blood transfusion, accidental needle stick, or exposure to patient blood during surgery.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV-1 infection and AIDS. The treatment involves administering to a patient in need of such treatment a pharmaceutical composition comprising a pharmaceutical carrier and a therapeutically effective amount of a compound of the present invention.

These pharmaceutical compositions may be in the form of orally administered suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions,may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

The compounds of this invention can be administered orally to humans in a dosage range of 1 to 100 mg/kg body weight in divided doses. A preferred dosage range is 1 to 10 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV-1 reverse transcriptase inhibitor compounds with one or more agents useful in the treatment of AIDS. For example, the compounds of this invention may be effectively administered, whether at periods of pre-exposure and/or post-exposure, in combination with effective amounts of the AIDS antivirals, immunomodulators, anti-infectives, or vaccines of Table II.

## TABLE II

### ANTIVIRALS

| Drug Name | Manufacturer | Indication |
|---|---|---|
| AL-721 | Ethigen (Los Angeles, CA) | ARC, PGL HIV-1 positive, AIDS |
| Recombinant Human Interferon Beta | Triton Biosciences (Almeda, CA) | AIDS, Kaposi's sarcoma, ARC |

| | | |
|---|---|---|
| Acemannan | Carrington Labs (Irving, TX) | ARC (See also immunomodulators) |
| Cytovene Ganciclovir | Syntex (Palo Alto, CA) | sight threatening CMV peripheral CMV retinitis |
| Drug Name | Manufacturer | Indication |
| d4T Didehydrodeoxythymidine | Bristol-Myers (New York, NY) | AIDS, ARC |
| ddI Dideoxyinosine | Bristol-Myers (New York, NY) | AIDS, ARC |
| EL10 | Elan Corp, PLC (Gainesville, GA) | HIV-1 infection (See also immunomodulators) |
| Foscarnet Trisodium Phosphonoformate | Astra Pharm. Products, Inc. (Westborough, MA) | CMV retinitis, HIV infection, other CMV infections |
| Dideoxycytidine; ddC | Hoffman-La Roche (Nutley, NJ) | AIDS, ARC |
| Novapren | Novaferon Labs, Inc. (Akron, OH) Diapren, Inc. (Roseville, MN, marketer) | HIV-1 inhibitor |

| Drug Name | Manufacturer | Indication |
|---|---|---|
| Peptide T Octapeptide Sequence | Peninsula Labs (Belmont, CA) | AIDS |
| Retrovir Zidovudine; AZT | Burroughs Wellcome (Rsch. Triangle Park, NC) | AIDS, adv, ARC pediatric AIDS, Kaposi's sarcoma, asymptomatic HIV infection, less severe HIV-1 disease, neurological involvement, in combination w/other therapies, post-exposure prophylaxis in health care workers |
| Rifabutin Ansamycin LM 427 | Adria Laboratories (Dublin, OH) Erbamont (Stamford, CT) | ARC |
| Dextran Sulfate | Ueno Fine Chem. Ind. Ltd. (Osaka, Japan) | AIDS, ARC, HIV positive asymptomatic |
| Virazole Ribavirin | Viratek/ICN (Costa Mesa, CA) | asymptomatic HIV positive, LAS, ARC |
| Alpha Interferon | Burroughs Wellcome (Rsch. Triangle Park, NC) | Kaposi's sarcoma, HIV in combination w/Retrovir |

IMMUNO-MODULATORS

| Drug Name | Manufacturer | Indication |
|---|---|---|
| Antibody which neutralizes pH labile alpha aberrant Interferon | Advanced Biotherapy Concepts (Rockville, MD) | AIDS, ARC |
| Drug Name | Manufacturer | Indication |
| in an immuno-adsorption column | | |
| AS-101 | Wyeth-Ayerst Labs. (Philadelphia, PA) | AIDS |
| Bropirimine | Upjohn (Kalamazoo, MI) | advanced AIDS |
| Acemannan | Carrington Labs, Inc. (Irving, TX) | AIDS, ARC (See also antivirals) |
| CL246,738 | American Cyanamid (Pearl River, NY) Lederle Labs (Wayne, NJ) | AIDS, Kaposi's sarcoma |
| EL10 | Elan Corp, PLC (Gainesville, GA) | HIV-1 infection (See also antivirals) |
| Gamma Interferon | Genentech (S. San Francisco, CA) | ARC, in combination w/TNF (tumor necrosis factor) |

| | | |
|---|---|---|
| Granulocyte Macrophage Colony Stimulating Factor | Genetics Institute (Cambridge, MA) Sandoz (East Hanover, NJ) | AIDS |

| Drug Name | Manufacturer | Indication |
|---|---|---|
| Granulocyte Macrophage Colony Stimulating Factor | Hoeschst-Roussel (Somerville, NJ) Immunex (Seattle, WA) | AIDS |
| Granulocyte Macrophage Colony Stimulating Factor | Schering-Plough (Madison, NJ) | AIDS<br>AIDS, in combination w/Retrovir |
| HIV-1 Core Particle ve HIV Immunostimulant | (Ft. Washington, PA) | Rorer seropositi |
| IL-2 Interleukin-2 | Cetus (Emerycille, CA) | AIDS, in combination w/Retrovir |
| IL-2 Interleukin-2 | Hoffman-La Roche (Nutley, NJ) | AIDS, ARC, HIV, in combination w/Retrovir |
| Immune Globulin Intravenous (human) | Cutter Biological (Berkeley, CA) | pediatric AIDS, in combination w/Retrovir |

| Drug Name | Manufacturer | Indication |
|---|---|---|
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute (Miami, FL) | AIDS, ARC |
| INTRON A Alpha-2 Interferon | Schering Plough (Madison, NJ) | Kaposi's sarcoma w/Retrovir: AIDS |
| Methionine-Enkephalin | TNI Pharmaceutical (Chicago, IL) | AIDS, ARC |
| MTP-PE Muramyl-Tripeptide | Ciba-Geigy Corp. (Summit, NJ) | Kaposi's sarcoma |
| Granulocyte Colony Stimulating Factor | Amgen (Thousand Oaks, CA) | AIDS, in combination w/Retrovir |
| rCD4 Recombinant Soluble Human CD4 | Genentech (S. San Francisco, CA) | AIDS, ARC |
| rCD4-IgG hybrids | | AIDS, ARC |

| | | |
|---|---|---|
| Recombinant Soluble Human CD4 | Biogen (Cambridge, MA) | AIDS, ARC |
| Roferon-A Interferon Alfa 2a | Hoffman-La Roche (Nutley, NJ) | Kaposi's sarcoma AIDS, ARC, in combination w/Retrovir |

| Drug Name | Manufacturer | Indication |
|---|---|---|
| SK&F106528 Soluble T4 | Smith, Kline & French Laboratories (Philadelphia, PA) | HIV-1 infection |
| Thymopentin | Immunobiology Research Institute (Annandale, NJ) | HIV-1 infection |
| Tumor Necrosis Factor; TNF | Genentech (S. San Francisco, CA) | ARC, in combination w/gamma Interferon |

ANTI-INFECTIVES

| Drug Name | Manufacturer | Indication |
|---|---|---|
| Clindamycin with Primaquine | Upjohn (Kalamazoo, MI) | PCP |
| Diflucan Fluconazole | Pfizer (New York, NY) | cryptococcal meningitis, candidiasis |

| | | |
|---|---|---|
| Pastille Nystatin Pastille | Squibb Corp. (Princeton, NJ) | prevention of oral candidiasis |
| Ornidyl Eflornithine | Merrell Dow (Cincinnati, OH) | PCP |
| Pentamidine Isethionate (IM & IV) | LyphoMed (Rosemont, IL) | PCP treatment |

| Drug Name | Manufacturer | Indication |
|---|---|---|
| Piritrexim | Burroughs Wellcome (Rsch. Triangle Park, NC) | PCP treatment |
| Pentamidine isethionate for inhalation | Fisons Corporation (Bedford, MA) | PCP prophylaxis |
| Spiramycin | Phone-Poulenc Pharmaceuticals (Princeton, NJ) | cryptosporidial diarrhea |
| Intraconazole-R51211 | Janssen Pharm. (Piscataway, NJ) | histoplasmosis; cryptococcal meningitis |
| Trimetrexate | Warner-Lambert | PCP |

OTHER

| Drug Name | Manufacturer | Indication |
|---|---|---|
| Recombinant Human Erythropoietin | Ortho Pharm. Corp. (Raritan, NJ) | severe anemia assoc. and Retrovir therapy |
| Megestrol Acetate | Bristol-Myers (New York, NY) | treatment of anorexia assoc. w/AIDS |

| Drug Name | Manufacturer | Indication |
|---|---|---|
| Total Enteral | Norwich Eaton Pharmaceuticals (Norwich, NY) | diarrhea and malabsorption related |

It will be understood that the scope of combinations of the compounds of this invention with AIDS antivirals, immunomodulators, anti-infectives or vaccines is not limited to the list in the above Table, but includes in principle any combination with any pharmaceutical composition useful for the treatment of AIDS.

Reverse Transcriptase Assay

The assay measures the incorporation of tritiated deoxyguanosine monophosphate by recombinant HIV reverse transcriptase (HIV $RT_R$) (or other RT) into acid-precipitable cDNA at the Km values of dGTP and poly r(C)·oligo $d(G)_{12-18}$. The inhibitors of the present invention inhibit this incorporation.

Thirty uL of a reaction mixture containing equal volumes of: 500 mM Tris·HCl (pH 8.2), 300 mM $MgCl_2$, 1200 mM KCl, 10 mM DTT, 400 μg/mL poly r(c)·oligo d(G) [prepared by dissolving 1.5 mg (25 U) poly r(C)·oligo d(G) in 1.5 ml sterile distilled $H_2O$ and diluting to 400 μg/ml], 0.1 μCi/μl [$^3$H] dGTP, 160 μM dGTP, was added to 10 μl sterile distilled $H_2O$, 2.5 μl of potential inhibitor and 10 μL of 5 nM purified HIV $RT_R$ in tubes. The mixture was incubated at 37°C for 45 minutes.

After incubation is complete, the tubes were cooled in ice for 5 minutes. Ice-cold 13% TCA containing 10 mM $NaPP_i$ (200 μl) are added and the mixture incubated on ice for 30 minutes. The precipitated cDNA is removed by filtration using presoaked glass filters [TCA, $NaPP_i$]. The precipitate is then washed with IN HCl, 10 mM $NaPP_i$.

The filter discs are then counted in a scintillation counter.

Under these conditions [dGTP] and poly r(C)·oligo $d(G)_{12-18}$ each are approximately equal to the appropriate Km value. Approximately 5-6,000 cpm of [$^3$H] GMP are incorporated into acid-precipitable material. The RT reaction is concentration- and time-dependent. DMSO (up to 5%) does not affect enzyme activity. Calculated $IC_{50}$ values for the compounds of this invention vary from about 10 nM to more than 300 μM.

Using the methodology described above, compounds of this invention were evaluated and found to exhibit reverse transcriptase inhibitory activity.

Example 1

Preparation of 4-ethyl-2-[(2-naphthylmethyl)amino]phenol:

A solution of 2-amino-4-ethylphenol(1.00 g. 7.28 mmol), 2-naphthaldehyde (1.13 g, 7.28 mmol), and p-toluenesulfonic acid (0.05 g) in methanol (50 mL) was stirred at room temp. for 24 h. To the resultant solution, sodium borohydride (0.82 g, 22 mmol) was added in small portions. After addition was completed, the mixture was stirred at room temperature for 30 min and concentrated under vacuum. The residue was then subjected to column chromatography on silica gel eluted with 10% ethyl acetate in hexane. Collection and concentration of appropriate fractions, followed by recrystallization (aqueous methanol) yielded 450 mg (22%) of analytically pure product.
Anal. Calc'd. for $C_{19}H_{19}NO$: C, 82.28; H, 6.9; N, 5.05
Found: C, 82.23; H, 6.97; N, 5.04.

EXAMPLE 2

Preparation of 4-ethyl-5-methyl-2-{[4,7-dichlorobenzo(b)furan-2-ylmethyl]amino}phenol

Step A: Preparation of 2,5-dichlorophenyl 2-chloroprop-2-enyl ether

A mixture of 2,5-dichlorophenol (41 g, 0.25 mol), 2,3-dichloropropene (28 g, 0.25 mol), and potassium carbonate (35 g, 0.25 mol) in acetone (100 mL) was refluxed overnight. The resultant slurry was filtered and the filtrate concentrated. The residue was dissolved in diethyl ether, washed two times with 1 M aq. sodium hydroxide and three times with brine. The ethereal solution was then dried over sodium sulfate, filtered and concentrated to yield 41 g (68%) of 2,5-dichlorophenyl 2-chloroprop-2-enyl ether.

Step B: Preparation of 2-(2-chloroprop-2-enyl)-3,6-dichlorophenol

A solution of 2,5-dichlorophenyl 2-chloroprop-2-enyl ether (10 g, 23 mmol) in 1,4-diisopropylbenzene (50 mL) was refluxed under an atmosphere of nitrogen for 48 h. The resultant mixture was then subjected to column chromatography eluted firstly with hexane, then with 2% dichloromethane in hexane. Collection and concentration of appropriate fractions yielded 8.7 g (87%) of 2-(2-chloroprop-2-enyl)-3,6-dichlorophenol.

Step C: Preparation of 2-(2-chloroprop-2-enyl)-3,6-dichlorophenyl acetate

A solution of 2-(2-chloroprop-2-enyl)-3,6-dichlorophenol (8.7 g, 37 mmol), acetic anhydride (4.5 g, 44 mmol), diisopropylethylamine (5.7 g, 44 mmol), and N,N-dimethylaminopyridine (0.45 g, 3.7 mmol) in-dichloromethane was stirred at room temperature overnight. The resultant dark brown solution was concentrated and the residue-subjected to column chromatography on silica gel eluted with 4% dichloromethane in hexane. Collection and concentration of appropriate fractions yielded 8.9 g (87%) of 2-(2-chloroprop-2-enyl)-3,6-dichlorophenyl acetate.

Step D: Preparation of 2-(3-chloropropan-2-one)-3,6-dichlorophenyl acetate

A solution of 2-(2-chloroprop-2-enyl)-3,6-dichlorophenyl acetate (8.9 g, 32 mmol) and m-chloroperbenzoic acid (80%, 10 g) in dichloromethane (85 mL) was stirred at room temperature for 8 days. The resultant mixture was then concentrated, and the residue was dissolved in ether. The ethereal solution was then washed sucessively with saturated aq. sodium bicarbonate, and brine. The organic solution was then dried over sodium sulfate and concentrated under vacuum to give the corresponding chloroepoxide.

Without further purification, the chloroepoxide was dissolved in dichloromethane and stirred under an atmosphere of hydrogen chloride gas at room temperature for 2 days. The resultant solution was concentrated to yield quantitatively 2-(3-chloropropan-2-one)-3,6-dichlorophenyl acetate.

Step E: Preparation of 2-iodomethyl-4,7-dichlorobenzo[b]furan

A suspension of 2-(3-chloropropan-2-one)-3,6-dichlorophenylacetate (2.0 g, 6.8 mmol) in conc. hydrochloric acid (50 mL) was heated at 90°C for 1 h. After cooling to room temperature, the white solid that precipitated out of solution was dissolved in chloroform. The organic solution was washed three times with brine, dried over sodium sulfate, filtered, and concentrated. The residue was then subjected to column chromatography on silica gel, eluted with chloroform:hexane, 1:1 v/v. Collection and concentration of appropriate fractions yielded 0.67 g (42%) of 2-chloromethyl-4,7-dichlorobenzo[b]furan. Treatment of the chloromethyl compound with sodium iodide in acetone at 60°C overnight yielded the corresponding 2-iodomethyl-4,7-dichlorobenzo[b]furan in 90% yield.

Step F: Preparation of 4,7-dichlorobenzol[b]furan-2-aldehyde

A solution of 2-iodomethyl-4,7-dichlorobenzo[b]furan (235 mg, 0.72 mmol) and anhydrous trimethylamine N-oxide (105 mg, 1.4 mmol) in anhydrous chloroform was heated at 50°C for 1.5 h. The resultant mixture was concentrated and the residue was subjected to column chromatography on silica gel eluted with chloroform. Concentration and collection of appropriate fractions yielded 69 mg (45%) of 4,7-dichlorobenzo[b]furan-2-aldehyde.

Step G: Preparation of 4-ethyl-5-methyl-2-{[4,7-dichlorobenzo[b]furan-2-yl]methyl}aminophenol

A solution of 2-amino-4-ethyl-5-methylphenol (49 mg, 0.32 mmol), 4,7-dichlorobenzo[b]furan-2-aldehyde (69 mg, 0.32 mmol), and p-toluenesulfonic acid (3 mg) in methanol (3 mL) was stirred at room temperature for 24 h. To the resultant solution, sodium borohydride (40 mg) was added in small portions. After addition was completed, the mixture was stirred at room temperature for an hour, and concentrated under vacuum. The residue was subjected to column chromatography on silica gel eluted with 3% methanol in chloroform. Collection and concentration of appropriate fractions, followed by recrystallization (aqueous methanol) yielded 24 mg (22%) of analytically pure product.
Anal. Calc'd. for $C_{18}H_{17}Cl_2NO_2$: C, 61.73; H, 4.89; N, 4.00.
Found: C, 61.74; H, 4.96; N, 4.38.

EXAMPLE 3

Preparation of 4-ethyl-2-{[3-(5-ethyl-2-methoxy-6-methylpyridyl)methyl]amino}phenol

A solution of 2-amino-4-ethylphenol (103 mg, 0.75 mmol), 5-ethyl-2-methoxy-6-methylpyridine-3-carboxaldehyde (134 mg, 0.75 mmol), and acetic acid (10 mg) in methanol (5 mL) was stirred at room temp. for 16 h. To the resultant yellow solution, sodium cyanoborohydride (0.12 g, 3.2 mmol) was added in small portions. After addition was completed, the mixture was stirred at room temperature for 30 min and concentrated under vacuum. The residue was subjected to column chromatography on silica gel eluted with 20% ethyl acetate in hexane. Collection and concentration of appropriate fractions, followed by recrystallization (hexane) yielded 34 mg (16% of analytically pure product.
Anal. Calc'd. for $C_{18}H_{24}N_2O_2$: C, 71.97; H, 8.05; N, 9.33.
Found: C, 71.68; H, 8.25; N, 9.02.

EXAMPLE 4

Preparation of 5-ethyl-3-{[3-(hydroxymethyl)anilino]methyl}-2-methoxy-6-methylpyridine

A solution of 3-aminobenzyl alcohol (250 mg, 2.03 mmol), 5-ethyl-2-methoxy-6-methylpyridine-3-carboxaldehyde (364 mg, 2.03 mmol), and acetic acid (10 mg) in methanol (4 mL) was stirred at room temp. for 16 h. To the resultant yellow solution, sodium borohydride was added in small portions until a clear colorless solution was obtained. The resultant mixture was stirred at room temp. for 30 min and concentrated under vacuum. The residue was dissolved in chloroform and washed with water. The organic layer was dried over anhydrous sodium sulfate, filtered, and concentrated. The product was then subjected to column chromatography on silica gel eluted with 5% methanol in chloroform. Collection and concentration of appropriate fractions yielded 190 mg (32.7%) of a pure viscous oil.

EXAMPLE 5

Preparation of 2-(4,5-dimethyl-2-methoxybenzylamino)-4-ethyl-5-methylphenol

A solution of 2-amino-4-ethyl-5-methylphenol (70 mg, 0.46 mmol), 2-methoxy-4,5-dimethylbenzaldehyde (75 mg, 0.46 mmol), and acetic acid (28 μL) in methanol (5 mL) was stirred at room temp. for 16 h. The yellow precipitate formed was solubilized by addition of chloroform (2 mL). To the resultant yellow solution, sodium cyanoborohydride was added in small portions until a clear colorless solution was obtained. The resultant mixture was stirred at room temp. for 30 min and concentrated under vacuum. The residue was subjected to column chromatography on silica gel eluted with 2% methanol in chloroform. Collection and concentration of appropriate fractions yielded 53 mg (38.5%) of analytically pure product.

Anal. Calc'd. for $Cl_{19}H_{25}NO_2$: C, 76.21; H, 8.41; N, 4.67.
Found: C, 75.90; H, 8.68; N, 4.61.

EXAMPLE 6

Preparation of 5-ethyl-2-methoxy-3-[(3-methoxy-anilino)methyl]-6-methylpyridine

To a suspension of sodium borohydride (21.3 mg, 0.7 mmol) in toluene (2.5 mL), acetic acid (149 μL) was added. The resultant mixture was stirred at room temperature for 30 min. Then *m*-anisidine (68.7 mg, 0.59 mmol), acetic acid (28 μL), and 5-ethyl-2-methoxy-6-methylpyridine-3-carboxaldehyde (100 mg, 0.59 mmol) were added in rapid succession. The resultant mixture was stirred at room temperature for 2.5 h, diluted with toluene (10 mL), and washed with brine. The organic layer was dried over anhydrous sodium sulfate, filtered, and concentrated. The residue was then subjected to column chromatography on silica gel eluted with 5% methanol in chloroform. Collection and concentration of appropriate fractions yielded 128 mg (83%) of a pure viscous oil.

EXAMPLE 7

Preparation of 5-ethyl-3-[(2-hydroxyanilino])methyl]-2-methoxy-6-methylpyridine

A solution of 2-aminophenol (250 mg, 2.29 mmol), 5-ethyl-2-methoxy-6-methylpyridine-3-carboxaldehyde (411 mg, 2.29 mmol), and acetic acid (20 mg) in methanol (4.5 mL) was stirred at room temp. for 16 h. To the resultant yellow solution, sodium cyanoborohydride was added in small portions until a clear colorless solution was obtained. The resultant mixture was stirred at room temp. for 1 h and concentrated under vacuum. The residue was dissolved in ethyl acetate and washed with water. The organic layer was dried over anhydrous sodium sulfate, filtered, and concentrated. The product was then subjected to column chromatography on silica gel eluted with 5% methanol in chloroform. Collection and concentration of appropriate fractions, followed by recrystallization (ethyl acetate-hexane) yielded 23 mg of a white solid.
Anal. Calc'd. for $C_{16}H_{20}N_2O_2 \cdot O_{.2}$ EtOAc: C, 69.58; H, 7.51; N, 9.66.
Found: C, 69.55; H, 7.23; N, 9.97.

EXAMPLE 8

Preparation of 5-ethyl-3-[(2-hydroxy-3-hydroxymethyl-5-methylanilino)methyl]-2-methoxy-6-methylpyridine

A solution of 2-hydroxy-3-hydroxymethyl-5-methylaniline (300 mg, 1.64 mmol), 5-ethyl-2-methoxy-6-methylpyridine-3-carboxaldehyde (290 mg, 1.62 mmol), and acetic acid (20 mg) in methanol (10 mL) was stirred at room temperature for 16 h. To the resultant yellow solution, sodium cyanoborohydride was added in small portions until a clear colorless solution was obtained. The resultant mixture was stirred at room temperature for 30 min and concentrated under vacuum. The residue was dissolved in ethyl acetate and washed with water. The organic layer was dried over anhydrous sodium sulfate, filtered, and concentrated. The product was then subjected to column chromatography on silica gel eluted with chloroform saturated with ammonia. Collection and concentration of appropriate fractions, followed by recrystallization (chloroform-hexane) yielded 265 mg of an off-white solid.
Anal. Calc'd. for $C_{18}H_{24}N_2O_3$: C, 68.33; H, 7.65; N, 8.85.
Found: C, 67.80; H, 7.57; N, 8.89.

While the foregoing specification teaches the principles of the present invention, with examples provided for the purpose of illustration, it will be understood that the practice of the invention encompasses all of the usual variations, adaptations, and modifications, as come within the scope of the following claims and its equivalents.

**Claims**

**1.** A Compound of the formula

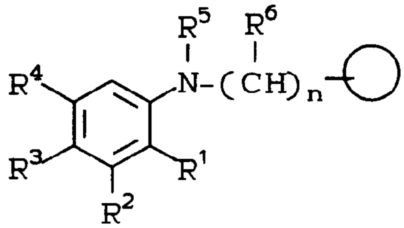

I

or a pharmaceutically acceptable salt thereof wherein:

n is 1-4;

$R^1$ is H, OH, or $CH_2OH$;

$R^2$ is H, or $CH_2OH$;

$R^3$ is H, $C_{1-8}$ alkyl unsubstituted or substituted with one or two of methoxy, or methylthio;

$R^4$ is

(i) $C_{1-8}$ alkyl, unsubstituted or substituted with one or two of $C_{1-3}$alkoxy, halo, or $C_{1-3}$ alkylthio;
(ii) $C_{1-3}$ alkylthio;
(iii) $C_{1-3}$ alkoxy; or
(iv) halo;
(v) hydroxy alkyl ($C_1$-$C_4$);

$R^5$ is H or $C_{1-2}$ alkyl;

$R^6$ is H or $C_{1-2}$ alkyl; and

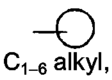 is aryl or heterocycle, each unsubstituted or substituted with one or more of

(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl, or
(j) halogen.

**2.** The compound of Claim 1 of the formula

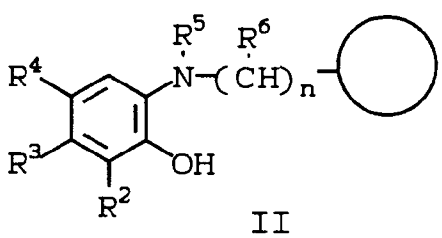

II

or a pharmaceutically acceptable salt thereof.

**3.** The compound of Claim 1 of the formula

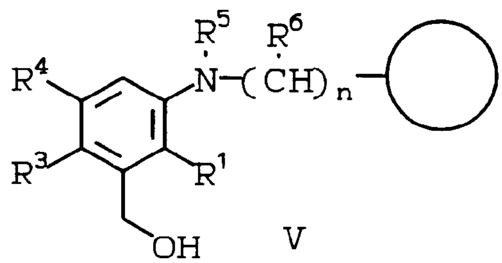

V

or a pharmaceutically acceptable salt thereof.

4. The compound of Claim 1, wherein
   $R^1$ is H, OH, or $CH_2OH$;
   $R^2$ is H or $CH_2OH$;
   $R^3$ is H or $CH_3$;
   $R^4$ is $C_{1-4}$ alkyl; and
aryl is
   (a) naphthyl, unsubstituted or substituted with one or more of $C_{1-6}$ alkyl, halogen, hydroxyl, or alkoxyl;
   (b) phenyl, unsubstituted or substituted with one or more of $C_{1-6}$ alkyl, halogen, hydroxyl, or alkoxyl; or
   (c) biphenyl.

5. The compound of Claim 4, wherein aryl is 2-naphthyl, 2-naphthyl substituted with methyl, 2-naphthyl substituted with chloro, 2-naphthyl substituted with methoxy; phenyl; 2-methoxyphenyl, 2-methoxyphenyl substituted with methyl, or 2-methoxyphenyl substituted with chloro.

6. The compound of Claim 5, wherein aryl is 2-naphthyl, phenyl or 2-methoxyphenyl.

7. The compound of Claim 1 wherein the heterocycle is thienyl, oxazolyl, thiazolyl, triazolyl, pyridyl, pyrazinyl, benzothienyl, quinolinyl, isoquinolinyl, benzimidazolyl, benzofuranyl, benzothiazolyl, benzoxazolyl unsubstituted or substituted with one or more of $C_{1-6}$ alkyl or halogen or hydroxyl or alkoxyl, naphth[3,2-d]oxazolyl, oxazolo[4,5-b]pyridyl, chromone, or benzopyrimidinone.

8. The compound of Claim 7 wherein the heterocycle is 2-oxazolyl, 2-pyridyl, 2-benzothienyl, 2-quinolinyl, 2-benzimidazolyl, 2-benzofuranyl, 2-benzothiazolyl, 2-oxazolo[4,5-b]pyridyl, or 2-benzoxazolyl unsubstituted or substituted with one or more of methyl, halogen, hydroxyl, or alkoxyl.

9. The compound of Claim 8 wherein the heterocycle is 2-benzoxazolyl, 7-methyl-2-benzoxazolyl, 4,7-dimethyl-2-benzoxazolyl, 7-chloro-2-benzoxazolyl, or 4,7-dichloro-2-benzoxazolyl.

10. The compound,
   4-ethyl-2-[(2-naphthylmethyl)amino]phenol;
   4-ethyl-5-methyl-2-{[4,7-dichlorobenzo(b)furan-2-ylmethyl]amino}phenol;
   4-ethyl-5-methyl-2-{[4,7-dimethylbenzo(b)furan-2-ylmethyl]amino}phenol;
   4-ethyl-2-{[3-(5-ethyl-2-methoxy-6-methyl-pyridyl)methyl]amino}phenol;
   5-ethyl-3-{[3-(5-ethyl-2-methoxy-6-methylpyridyl)methyl]amino}benzyl alcohol;
   5-ethyl-3{[3-(hydroxymethyl)anilino]methyl}-2-methoxy-6-methylpyridine;
   2-(4,5-dimethyl-2-methoxybenzylamino)-4-ethyl-5-methylphenol;
   5-ethyl-2-methoxy-3[(3-methoxyanilino)methyl]-6-methylpyridine;
   5-ethyl-3-[(2-hydroxyanilino)methyl]-2-methoxy-6-methylpyridine; or
   5-ethyl-3-[(2-hydroxy-3-hydroxymethyl-5-methylanilino)methyl]-2-methoxy-6-methylpyridine.

11. The compound
   4-ethyl-2-{[3-(5-ethyl-2-methoxy-6-methyl-pyridyl)methyl]amino}phenol.

12. The use of a compound of Claim 1 for the manufacture of a medicament for inhibiting HIV-1 reverse transcriptase.

**13.** The use of a compound of Claim 1 for the manufacture of a medicament for preventing infection by HIV-1, or of treating infection by HIV-1 or of treating AIDS or ARC.

**14.** A pharmaceutical composition useful for inhibiting HIV-1 reverde transcriptade, comprising an effective amount of a compound of Claim 1 and a pharmaceutically acceptable carrier.

**15.** A pharmaceutical composition useful for preventing or treating infection by HIV-1 or for treating AIDS or ARC, comprising an effective amount of a compound of Claim 1 and a pharmaceutically acceptable carrier.

European Patent Office

# PARTIAL EUROPEAN SEARCH REPORT

which under Rule 45 of the European Patent Convention shall be considered, for the purposes of subsequent proceedings, as the European search report

Application Number: EP 92 30 2531

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 100, no. 26, 25th June 1984, page 678, abstract no. 220539f, Columbus, Ohio, US; J. CSASZAR: "Spectroscopic study of reduction products of aromatic Schiff bases and of their copper complexes", & ACTA PHYS. CHEM. 1983, 29(3-4), 139-44<br>* Abstract *<br>--- | 1,4 | C 07 D 213/64<br>C 07 D 307/81<br>C 07 C 211/48<br>A 61 K 31/44<br>A 61 K 31/34 |
| X | CHIMICA THERAPEUTICA, vol. 5, no. 3, 1970, pages 169-184, FR; M. DESCAMPS et al.: "Recherches dans la série des benzofurannes. XLII. Synthèse de benzofuryl-2 méthylamines et d'amides d'acides coumaryliques"<br>* Compounds 63,65,68,69; table VII *<br>--- -/- | 1,7,8 | |

TECHNICAL FIELDS SEARCHED (Int. Cl.5)

C 07 D
C 07 C
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely: 6,10,11
Claims searched incompletely: 1-5,7-9,12-15
Claims not searched:
Reason for the limitation of the search:

The subject matter of the present application is so broad that a complete search is not possible on economic grounds. (Guidelines B III.3.2). Therefore the search has been limited to the examples and the claims as indicated below (Rule 45 EPC).

| Place of search | Date of completion of the search | Examiner |
|---|---|---|
| THE HAGUE | 09-07-1992 | BOSMA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number
EP 92 30 2531

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| L | JOURNAL OF MEDICINAL CHEMISTRY, vol. 34, no. 9, September 1991, pages 2922-2925, Washington, DC, US; W.S. SAARI et al.: "2-Pyridinone derivatives: a new class of nonnucleoside, HIV-1-specific reverse transcriptase inhibitors" * Whole document * ----- | 1-15 | |
| | | | TECHNICAL FIELDS SEARCHED (Int. Cl.5) |

EPO FORM 1503 03.82 (P0410)